**Europäisches Patentamt**

**European Patent Office**

**Office européen des brevets**

(19)

(11) Numéro de publication: **0 128 783**
**B1**

(12) **FASCICULE DE BREVET EUROPEEN**

(45) Date de publication du fascicule du brevet:
**24.06.87**

(51) Int. Cl.⁴: **A 61 H 5/00,** A 61 B 3/04

(21) Numéro de dépôt: **84400414.3**

(22) Date de dépôt: **01.03.84**

(54) Instrument pour l'examen, la mesure et le traitement des anomalies de la vision binoculaire.

(30) Priorité: **14.06.83 FR 8309823**

(43) Date de publication de la demande:
**19.12.84 Bulletin 84/51**

(45) Mention de la délivrance du brevet:
**24.06.87 Bulletin 87/26**

(84) Etats contractants désignés:
**AT BE CH DE GB IT LI LU NL SE**

(56) Documents cités:
**FR - A - 727 997**
**GB - A - 388 200**
**GB - A - 425 474**
**GB - A - 442 956**
**US - A - 2 213 467**
**US - A - 2 706 981**
**US - A - 3 972 319**

(73) Titulaire: **Mawas, Lucie Jacqueline, 5 ter Avenue du Général Sarrail, F-78400 Chatou (FR)**

(72) Inventeur: **Mawas, Lucie Jacqueline, 5 ter Avenue du Général Sarrail, F-78400 Chatou (FR)**

(74) Mandataire: **Picard, Jean-Claude Georges et al, Cabinet Plasseraud 84, rue d'Amsterdam, F-75009 Paris (FR)**

## Description

La présente invention concerne un instrument pour l'examen, la mesure et le traitement des anomalies de la vision binoculaire, du type comprenant une monture pouvant être analogue à celle de lunettes classiques, comportant un pont central propre à prendre assise sur le nez du sujet et reliant deux supports de verres optiques agencés pour maintenir ceux-ci respectivement en face des yeux, sur ces supports étant articulées, également de façon connue, deux branches propres, lorsqu'elles sont dépliées, à encadrer la tête du sujet en étant supportées par ses oreilles (FR-A-727 997).

Parmi les instruments destinés à l'examen et à la mesure des anomalies de la vision binoculaire, on connaît déjà, par exemple, l'instrument dit «déviomètre de LAVAT», lequel comprend une tige inclinée portant deux points lumineux et mobile en rotation sur une autre tige verticale fixe, portée par un socle. Le sujet est placé en face de l'appareil, sa tête étant maintenue immobile par un appui-tête; il doit fixer l'un ou l'autre des deux points lumineux. La déviation est étudiée au test dit «de l'écran» à l'aide de prismes maintenus devant les yeux du sujet par l'examinateur, dont une main est ainsi constamment consacrée à cette tâche.

Il s'agit là, pour toutes ces raisons, d'un appareil lourd, encombrant et coûteux, d'une utilisation relativement malcommode, dont le fonctionnement est en outre subordonné à l'accès à une alimentation en courant électrique. Le sujet ne peut être examiné qu'au cabinet du praticien et doit donc se déplacer.

Quant au synoptophore et au synoptomètre, il s'agit encore d'instruments lourds, encombrants, coûteux et d'un usage malcommode, dont le principe est d'annuler la déviation phorique ou tropique des deux yeux, à l'aide de bras mobiles porteurs de prismes et de miroirs. Là également, l'examen ne peut se faire qu'en milieu spécialisé.

Pour ce qui concerne maintenant, non plus les appareils ou instruments d'examen et de mesure des anomalies de la vision binoculaire, mais les appareils ou instruments de traitement de ces anomalies, on connaît par exemple l'appareil dit «de Javal», lequel permet, par des exercices particuliers, une lecture contrôlée d'un texte et une rééducation progressive permettant de traiter le strabisme.

Cet appareil comporte une barre portant, à une de ses extrémités, un support pour le texte à lire et, à son autre extrémité, un appui-tête; entre les deux est fixée une barre verticale opaque, et l'exercice consiste, pour le sujet, à lire le texte sans aucun mouvement de la tête, d'une manière continue, sans qu'aucune lettre du texte ne lui apparaisse cachée par l'obstacle.

Il s'agit là encore d'un appareil qui, malgré sa relative simplicité, est d'une utilisation malcommode et ne permet pas d'obtenir une très grande efficacité, surtour lorsqu'il s'agit de traiter des enfants, lesquels, par jeu, tentent de deviner les lettres cachées ou ont tendance à déplacer le texte pour lire ces lettres.

Par le document US-A-2 706 981 on connaît un appareil destiné à exercer les muscles oculaires et comportant un rotor pouvant à la fois pivoter et coulisser sur une tige dont une extrémité est supportée par le nez du patient, mais il s'agit là uniquement d'un appareil d'exercice des muscles et non pas à proprement parler d'un appareil permettant entre autres une mesure des anomalies de la vision binoculaire.

Le but de la présente invention est de remédier à tous les inconvénients des appareils ou instruments connus, des types ci-dessus rappelés, et elle a donc pour objet la réalisation d'un instrument multi-fonctions pour l'examen, la mesure et le traitement des anomalies diverses de la vision binoculaire, instrument qui puisse être porté par le sujet et qui soit donc léger, tout en étant de réalisation simple et peu coûteuse.

A cet effet, un instrument conforme à l'invention est caractérisé en ce que ladite tige principale est montée de façon rotative sur ledit pont de la monture, de sorte à pouvoir prendre diverses positions d'inclinaison pas rapport à l'horizontale, au moins dans le plan vertical sagittal du sujet, et éventuellement dans des plans verticaux inclinés latéralement d'un côté ou de l'autre de ce plan sagittal.

On constate qu'il s'agit effectivement d'un instrument très simple, qui peut être peu coûteux et qui, étant porté par le sujet, laissera libres les mains de celui-ci, ainsi que les mains du praticien, notamment pour le montage des différents verres optiques sur la monture et pour la notation de ses mesures.

La définition fournie ci-dessus d'un instrument conforme à l'invention permettra d'effectuer certains exercices.

Il pourra s'agir notamment d'exercices de lecture effectués avec une tige principale de longueur prescrite et adaptée au sujet concerné; l'extrémité de cette tige constituera le lieu, désiré par le praticien, du croisement lors de la lecture, c'est-à-dire le lieu désiré de la fixation binoculaire centrale. La longueur de la tige principale permettra pratiquement le maintien d'une bonne statique cervico-dorsale du sujet, la distance entre les yeux et l'extrémité distale de la tige déterminant l'accommodation requise.

Des ajouts différents faits à l'instrument qui vient d'être défini pourront permettre d'effectuer d'autres opérations plus complexes, comme cela sera mieux vu à la lecture des exemples qui seront donnés plus bas avec référence aux figures du dessin annexé.

On pourra prévoir par exemple que ledit pont de la monture porte une alidade et/ou un cercle ou arc de cercle gradué, dispositifs propres à permettre d'effectuer une mesure des différents angles d'inclinaison de ladite tige principale dans ledit plan sagittal, ou de l'angle d'écartement latéral du plan vertical qui la contient, par rapport à ce même plan sagittal.

---

Un instrument conforme à l'invention, réalisé dans l'un ou l'autre des différents modes d'exécution ci-dessus définis, pourra encore avantageusement être caractérisé en ce qu'une règle opaque, en principe dirigée vers le bas, est montée coulissante sur ladite tige principale. Un instrument ainsi réalisé permettra, notamment dans le cas d'une tige principale fixe, d'effectuer des exercices de lecture contrôlée sans utiliser l'appareil de Javal.

On comprend en effet que ladite règle dirigée vers le bas remplacera la barre verticale opaque de l'appareil de Javal, en s'interposant successivement entre les deux yeux du sujet et le texte au cours de la lecture, sans que le sujet puisse en quelque sorte tricher, puisque le déplacement de sa tête, laquelle porte l'instrument, n'aura plus aucun effet.

Quel que soit le mode de réalisation choisi, il sera toujours avantageux de prévoir, et là encore dans des buts de mesure, de contrôle ou d'exercices qui seront indiqués plus bas, qu'au moins un curseur de repère est monté coulissant sur ladite tige principale.

On pourra également prévoir qu'un miroir est monté coulissant sur ladite tige principale, ceci pour augmenter la longueur apparente de la tige principale et permettre d'effectuer des mesures de convergence et de divergence relatives, ainsi que de réaliser aisément la diplopie physiologique.

Notamment dans le cas où il est équipé d'un curseur, il pourra également être intéressant de prévoir, sur l'appareil, une tige principale graduée, ce qui permettra de repérer la distance entre les objets qui y sont montés coulissants et la monture.

Un instrument conforme à l'invention pourra encore se caractériser en ce que sur l'extrémité distale de la tige principale est montée une tige secondaire de préférence graduée, s'étendant dans une direction faisant un angle déterminé avec la direction de ladite tige principale.

La fixation par le sujet de l'extrémité libre de cette tige secondaire permettra par exemple d'étudier la déviation de ses yeux grâce au test dit «de l'écran».

Par ailleurs, en déplaçant un curseur le long de cette seconde tige, on pourra encore vérifier si le sujet maintient le dédoublement de la tige principale lorsque sa fixation varie latéralement.

A cet effet, on prévoit que ladite tige secondaire est montée sur l'extrémité distale de la tige principale de sorte à pouvoir effectuer une rotation d'angle déterminé, axée sur cette dernière.

Il sera aussi intéressant de prévoir qu'un anneau gradué ou analogue est fixé à l'extrémité proximale ou distale de ladite tige principale, pour permettre de mesurer l'angle que fait la direction de ladite tige secondaire avec le plan vertical contenant ladite tige principale.

Pour favoriser la fixation par les yeux du sujet de l'extrémité libre de ladite tige secondaire, il sera également intéressant de prévoir qu'un objet de repère avantageusement lumineux, est monté à l'extrémité libre de ladite tige secondaire.

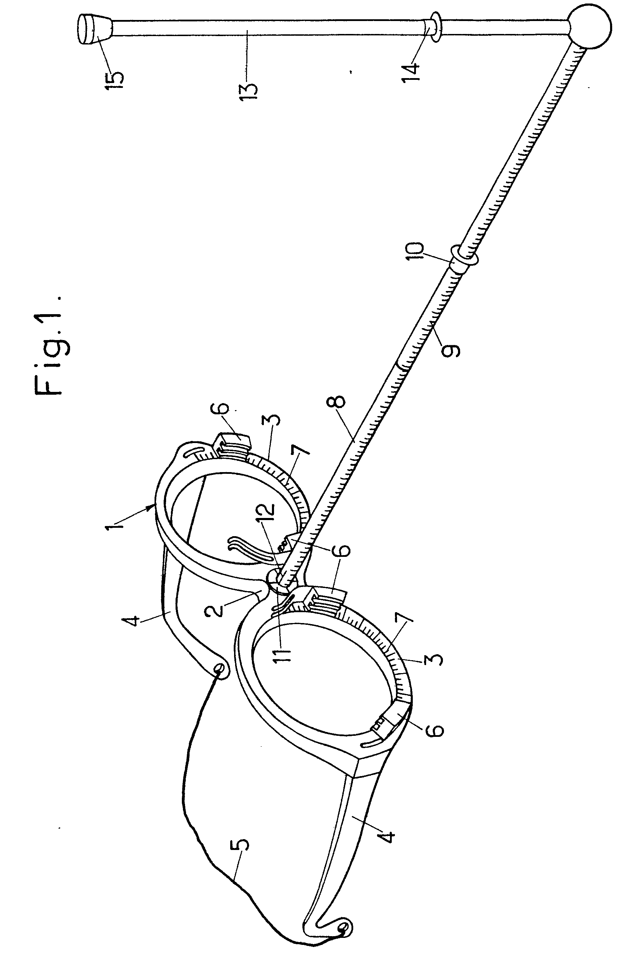

Quatre modes d'exécution d'un instrument conforme à la présente invention vont maintenant être décrits à titre d'exemples nullement limitatifs, avec référence aux figures 1 à 4, du dessin ci-annexé.

Sur la figure 1, on a représenté schématiquement une paire de lunettes comprenant une monture 1 analogue à celle de lunettes classiques, comportant un pont central 2 propre à prendre assise sur le nez du sujet et reliant deux supports 3 de verres optiques (non représentés), sur ces supports 3 étant articulées, également de façon connue, deux branches 4 propres, lorsqu'elles sont dépliées, à encadrer la tête de ce sujet en étant supportées par ses oreilles. Un élastique 5 fixé aux extrémités des branches 4 peut faire le tour de la tête pour parfaire l'assujettissement de cet instrument devant les yeux du sujet. A proximité du pont 2, les deux supports 3 comportent des arceaux à rainures 6 permettant de fixer devant les cerclages que forment les supports 3 différents verres optiques, notamment des verres correcteurs (de préférence non bagués), des verres colorés ou encore un prisme dit «de Risley» ou des verres striés dits «de Bagolini». Ces arceaux à rainures peuvent également supporter, de façon connue, des prismes simples ou de Fresnel.

La monture 1 peut être à écart pupillaire variable ou fixe, auquel cas on prévoira une série de montures à écarts pupillaires différents, par exemple de 55, 60 et 63 mm. La monture porte avantageusement une graduation de type TABO de 0 à 180°, laquelle a été référencée 7 pour chacun des supports 3.

Conformément à l'invention, une tige principale 8 est fixée sur le pont médian 2 de façon à occuper en principe le plan sagittal du corps du sujet lorsque celui-ci porte l'instrument, c'est-à-dire le plan perpendiculaire à la ligne des deux yeux. Cette tige principale 8 est mobile et porte de préférence une graduation linéaire 9 et un ou plusieurs curseurs tels que celui qui a été référencé en 10. En outre, une bague graduée sur 360°, référencée en 11, est fixée au pont médian 2 et permet, en coopération avec un repère fixe 12 de l'emplanture de la tige 8, de repérer l'orientation de cette dernière autour de son axe. Pour fixer les idées, on peut mentionner encore que la longueur de la tige principale 8 pourra être de l'ordre de 20 cm environ.

A l'extrémité distale de la tige principale 8 est fixée à angle droit une tige secondaire 13, cette tige pouvant donc s'étendre dans différentes directions lors de la rotation de la tige principale 8 autour de son axe. Cette orientation de la tige secondaire 13 par rapport au plan sagittal pourra donc être repérée par la graduation de la bague ou anneau 11 qui se trouvera en face du repère fixe 12 de la tige 8.

De préférence, la tige secondaire 13, comme la tige principale 8, porte un ou plusieurs curseurs tels que celui qui a été représenté sous la forme d'un anneau coulissant 14.

La tige secondaire peut également porter une graduation linéaire (non représentée) et est de longueur variable, cette longueur étant en pratique de 30 cm.

Enfin, pour mieux attirer l'attention du sujet examiné, on a prévu de fixer à l'extrémité libre de la tige secondaire 13, un objet brillant ou lumineux, lequel a été référencé en 15; il peut s'agir à la rigueur simplement d'un objet peint d'une couleur vive.

Ceci étant, on pourra, à l'aide d'un tel instrument, réalisé conformément à ce premier mode d'exécution de l'invention, faire un certain nombre d'examens, de mesures et d'exercices, ces examens pouvant globalement être conduits d'une façon assez semblable à ceux auxquels on procédait auparavant à l'aide des instruments ou appareils connus de la technique antérieure, évoqués au début.

On pourra par exemple procéder à un examen de la motilité dans les neuf positions princiales du regard. Le sujet fixe alors l'extrémité 15 de la tige secondaire 13, et l'on étudie la déviation de ses yeux grâce au test de l'écran. L'examinateur couvre alternativement les deux yeux, et le mouvement que fait un œil pour rattraper le point de fixation est annulé par un prisme glissé sur l'un des arceaux de support 6, la puissance de ce prisme fournissant la mesure de l'amplitude de la déviation.

Dans un autre type d'utilisation, on pourra faire varier la position du curseur 14 le long de la tige secondaire 13 et étudier ainsi la poursuite des yeux du sujet. En effectuant un tel déplacement du curseur 14 le long de la tige 13, un peut également vérifier si le sujet maintient bien le dédoublement de la tige principale 8, lorsque sa fixation varie latéralement, s'agissant ici d'un examen cependant subjectif.

L'étude peut éventuellement se compléter par celle des reflets cornéens dans les positions choisies, tous ces examens se faisant de toute façon avec un minimum d'interpositions manuelles.

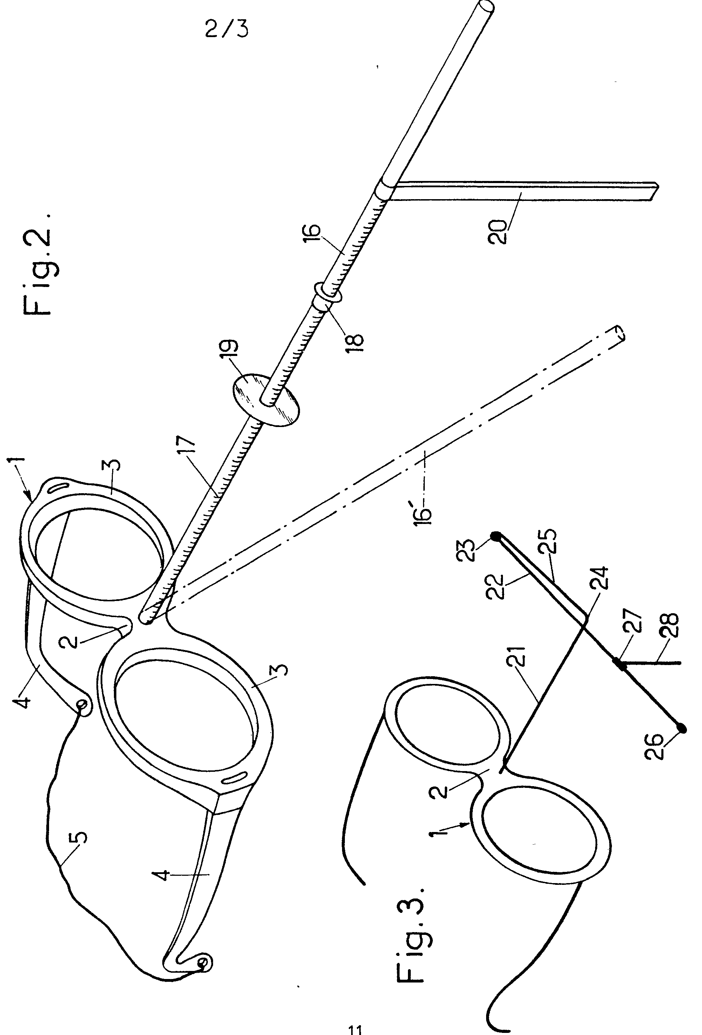

Dans le mode d'exécution de la figure 2, dans laquelle on a utilisé les mêmes référence pour repérer les différentes parties de la monture 1 (pont, supports de verres optiques, branches et élastique), une tige principale référencée 16 a été fixée sur le pont 2. Cette tige est de préférence graduée, comme référencé en 17, et peut se plier ou s'orienter par pivotement dans différentes directions du plan sagittal, comme ceci a été indiqué par la référence 16' repérant, la psoition indiquée en pointillé. Cette tige principale 16 peut encore porter un curseur tel que celui qui a été référencé en 18, et éventuellement un miroir tel que celui qui a été référencé en 19, ceci permettant d'en augmenter la longueur apparente.

Conformément à cet autre mode d'exécution de l'invention, on a prévu de monter, coulissante sur la tige 16, un règle opaque 20 dirigée vers le bas et en principe située dans le plan sagittal du sujet. Cette tige 20, comme indiqué plus haut, permettra de faire effectuer à ce sujet des exercices de lecture contrôlée sans utiliser l'appareil de Javal.

Grâce à cet instrument, on peut effectuer d'autres examens, et notamment l'examen et l'étude de l'état de la vision binoculaire de près et de loin. La tige principale 16 peut supporter des cibles (à la place du miroir 19) et permettre de réaliser aisément la diplopie physiologique. En cas de vision binoculaire, la tige 16 est vue sous la forme de deux tiges qui se croisent, sont concourantes ou parallèles selon l'endroit où est situé le point de fixation et selon l'existence d'une hétérophorie ou d'une hétérotropie. L'image de la tige vue à droite représente l'œil gauche et l'image de la tige vue à gauche représente l'œil droit.

On pourra également grâce à cet instrument de la figure 2, pratiquer sur le sujet une méthode de la rééducation de la vision binoculaire, lors de la mise en œuvre de laquelle il pourra avoir les mains libres et donc vaquer à ses occupations habituelles à domicile, et en particulier lire, écrire, coudre, regarder la télévision, etc., tout en rééduquant inconsciemment sa fixation binoculaire, sa convergence et son accommodation.

On pourra également effectuer un contrôle constant de la participation des deux yeux, soit seulement de la rétine périphérique, soit de la rétine centrale.

On pourra également, comme ceci a déjà été mentionné, entreprendre une rééducation de la statique corporelle, et en particulier de la colonne vertébrale lors de la lecture, tant chez le jeune myope dont le défaut principal est de lire de trop près que chez le presbyte dont le défaut principal est de lire de trop loin.

On pourra également éduquer le presbyte et l'entraîner à bien placer sa tête pour profiter au mieux des verres à focale variable. La tige étant légèrement inclinée vers le bas oblige le sujet à relever le menton et à regarder par le bas du verre s'il veut voir le croisement des axes sur le texte lu.

Chez la personne frappée de strabisme, cet instrument de la figure 2 permettra de trouver le lieu du croisement des axes subjectivement. Si la mesure doit être objective, l'objet fixé va être lumineux; il doit être placé là où les reflets cornéens sont centrés. On constate l'obtention du lieu de croisement des axes visuels si aucun mouvement ne se produit au test de l'écran.

Outre le miroir 19 représenté à la figure 2, l'instrument de ce mode d'exécution de l'invention peut aussi porter toutes sortes d'objets et par exemple des stéréogrammes à écartes variables, le porte-stéréogramme portant également une graduation, de telles dispositions permettant de s'assurer de la bonne perception du relief.

Un instrument conforme au mode d'exécution de la figure 2 pourra également servir à un traitement, la tige ayant une longeur prescrite et adaptée au sujet concerné, cette longueur déterminant la convergence nécessaire pour être en bifixation. Dans ce cas, il sera cependant possible de modifier cette convergence en plaçant un anneau ou analogue à une distance choisie, par exemple à l'endroit auquel, sur la figure 2, on a représenté le miroir 19. Par exemple, si la tige mesure 33 cm et si le sujet en fixe l'extrémité et obtient le croisement apparent des tiges dans le plan du texte lu, il développera trois dioptries d'accommodation et trois angles métriques de convergence.

Si maintenant, l'anneau ou analogue est coulissé sur la tige 16 à 20 cm des yeux et si l'on demande au sujet de fixer cet anneau en même temps qu'il lit, le corisement lui apparaîtra en avant du plan de lecture, et il convergera alors de cinq angles métriques, toujours pour trois dioptries d'accommodation.

L'orthoptiste ou l'ophtalmologiste ayant déjà pratiqué une étude de la motilité et des problèmes sensoriels du sujet prescrira alors sans difficulté le verre ou le prisme le plus faible, capable d'obtenir la déneutralisation de l'œil.

Le port de l'instrument (complété par sa tige) à domicile constituera à lui seul un exercice qui pourra aider à la prise de conscience et au maintien de cette prise de conscience de la participation de l'œil le plus faible dans l'acte visuel. Cet exercice pourra être majoré par des «pénalisations» et par la mobilisation d'objets sur la tige, ces «pénalisations» étant des verres inadéquats avec lesquels on brouille l'œil le meilleur afin de permettre à l'œil le plus faibel (amblyope ou dominé) de participer à la vision binoculaire.

En résumé, in instrument conforme à la présente invention permettra, selon le mode d'exécution choisi, de procéder à des examens, mesures et exercices de rééducation dans le domaine des anomalies de la vision binoculaire, et d'une façon générale de procéder à l'étude des rapports entre l'accommodation et la convergence, ainsi qu'à la rééducation de la position de lecture optimale chez les myopes et les presbytes, cette rééducation affectant la position de lecture à bonne distance et la statique correcte du dos.

A la figure 3 on a représenté un troisième mode d'exécution possible de l'invention, dans lequel la monture 1 de la paire de lunettes est équipée d'une tige principale, de préférence pivotante 21, fixée sur le pontet 2, et d'une tige secondaire 22 s'étendant par exemple dans un plan perpendiculaire à la tige 21 et qui est reliée par l'une de ses extrémités 23 à l'extrémité distale 24 de la tige principale 21 par un bras 25. En d'autres termes, la tige secondaire 22 s'étend de part et d'autre du plan contenant la tige 21 et qui lui est perpendiculaire.

Aux extrémités 23 et 26 de la tige secondaire 22 sont prévus des points, là encore destinés à attirer le regard du sujet, et qui peuvent être constitués de points lumineux (diodes lunieuses) ou de boules ou analogues, de couleur vive. Enfin, un curseur 27 est monté coulissant sur la tige secondaire 22.

Ce curseur permet ainsi d'étudier le mouvement de poursuite des yeux du sujet dans toutes les directions du regard, d'une extrémité à l'autre de la tige, sans aucune interruption et quelle que soit l'orientation de cette tige 22 dans l'espace, puisqu'on peut la faire pivoter par rotation de la tige principale 21 sur le pontet 2.

L'instrument qui vient d'être décrit permet également d'étudier le mouvement saccadé des yeux, passant rapidement de l'observation de l'extrémité 23 à l'observation de l'extrémité 26 et inversement.

Sur cette figure, on n'a pas représenté les moyens de mesure de l'angle de rotation de la tige 21 sur le pontet 2, mais il est bien entendu qu'ils pourraient être de tout type connu et être constitués par exemple de la même manière que dans le mode d'exécution de la figure 1. Le bras 25 pourrait également être monté pivotant sur l'extrémité distale 24 de la tige principale 21, auquel cas cette dernière serait montée fixe sur le pontet 2.

De même, il est bien entendu que la tige secondaire 22 pourrait porter une graduation entre ses deux extrémités 23 et 26, ceci permettant de repérer exactement la position du curseur 27.

En 28, on a représenté une tige de manipulation reliée au curseur 27 et que l'examinateur ou le sujet peut saisir par son extrémité inférieure pour manipuler ce curseur, ceci évite la gêne que pourraient causer les doigts manipulant directement ce curseur 27. Une telle tige de manipulation pourrait également équiper les curseurs des modes d'exécution des figures 1 et 2.

Quel que soit le mode d'exécution choisi, il sera avantageux de constituer les différentes tiges et accessoires d'un instrument conforme à l'invention en matériau assez léger, pour éviter toute gêne au sujet, notamment lorsque, pour effectuer des exercices à domicile, il devra porter l'instrument pendant des temps relativement longs.

De même, il est bien entendu que les différents moyens de mesure d'angle ou de distance pourront être constitués de la manière la plus appropriée et que, par exemple, les graduations permettant de repérer les rotations des tiges ou l'orientation des verres optiques sur la monture pourront être effectuées en grades ou en degrés.

Il est à noter que les différents curseurs, par exemple le curseur 10 du mode d'exécution de la figure 1, qui est représenté sous la forme d'une espèce de rivet, pourraient être remplacés par tout autre moyen ayant le même objet, et par exemple notamment par une bulle d'air dans le cas où l'on prévoirait une tige principale 8 creuse transparente et emplie d'un liquide, par exemple d'un liquide coloré. Ceci faciliterait grandement l'exercice, puisque le sujet pourrait très commodément faire varier le positionnement de la bulle-curseur le long de la tige principale 8 en modifiant simplement l'inclinaison de sa tête.

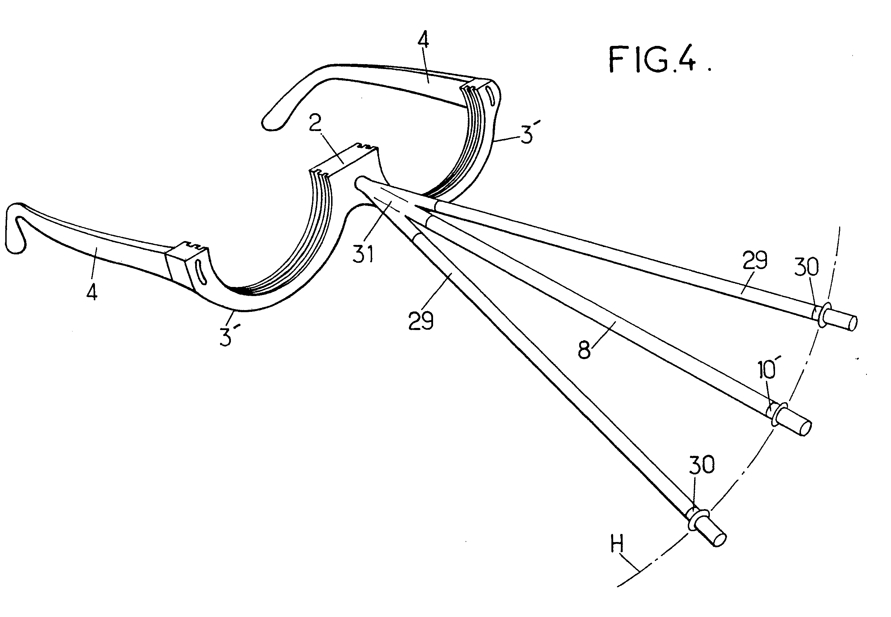

Sur la figure 4, on a représenté encore un autre mode d'exécution de l'invention, dans lequel une tige principale 8, montée sur une embase tripode 31 du pont central 2 de la monture, est associée, à deux autres tiges 29, ces tiges étant fixées également à l'embase 31 et s'étendant de part et d'autre de la tige principale 8. Une embase appropriée permettra de rendre la tige principale 8 et les deux tiges accessoires 29 orientables dans différentes directions.

La tige principale 8 peut porter un repère mobile 10' et les tiges accessoires 29 un repère du même genre, référencé 30.

Les tiges accessoires 29 permettent de mettre en évidence la diplopie physiologique périphérique. Le sujet qui fixe le repère 10' voit se dédoubler la tige principale 8, ainsi que les tiges accessoires 29.

Les trois repères 10' et 30 sont vus simples et figurent l'horoptère, lequel a été représenté sur la figure 4 par un arc de cercle tireté H. Le repère 10' représente la vision simultanée centrale et les repères 30 la vision simultanée périphérique.

Si le sujet fait se déplacer les repères 30 en maintenant le regard fixé sur le repère 10', il stimule sa vision périphérique en voyant les tiges accessoires 29 se déplacer.

On observe en outre sur la figure 4 que la monture est modifiée par rapport à celle des figures précédentes 1 à 3, les supports 3 étant en forme de demi-cercles ouverts vers le haut et étant pourvus intérieurement de rainures, par exemple au nombre de 3, ce qui permet plus simplement l'utilisation de verres correcteurs ou de verres spéciaux, prismes ou autres.

## Revendications

1. Instrument pour l'examen, la mesure et le traitement des anomalies de la vision binoculaire, du type comprenant une monture (1) pouvant être analogue à celle de lunettes classiques, comportant un pont central (2) propre à prendre assise sur le nez du sujet et reliant deux supports (3) de verres optiques agencés pour maintenir ceux-ci respectivement en face des yeux, sur ces supports (3) étant articulées, également de façon connue, deux branches (4) propres, lorsqu'elles sont dépliées, à encadrer la tête du sujet en étant supportées par ses oreilles, caractérisé en ce que sur la face antérieure dudit pont (2) est fixée une tige principale (8, 16) s'étendant vers l'avant, à savoir dans une direction générale opposée à celle desdites branches (4) lorsqu'elles sont en position dépliée, cette tige ayant une longueur suffisante pour provoquer la diplopie physiologique, et en ce que ladite tige principale (8, 16) est montée de façon rotative sur ledit pont (2) de la monture (1), de sorte à pouvoir prendre diverses positions d'inclinaison par rapport à l'horizontale, au moins dans le plan vertical sagittal du sujet, et éventuellement dans des plans verticaux inclinés latéralement d'un côté ou de l'autre de ce plan sagittal.

2. Instrument selon la revendication 1, caractérisé en ce que la monture (1) porte une alidade et/ou un cercle ou arc de cercle gradué (11), dispositifs propres à permettre d'effectuer une mesure des différentes angles d'inclinaison de ladite tige principale (8, 16) dans ledit plan sagittal, ou de l'angle d'écartement latéral du plan vertical qui la contient, par rapport à ce même plan sagittal.

3. Instrument selon la revendication 1 ou 2, caractérisé en ce qu'une règle opaque (20), en principe dirigée vers le bas, est montée coulissante sur ladite tige principale (8, 16).

4. Instrument selon l'une quelconque des revendications précédentes, caractérisé en ce qu'au moins un curseur de repère (10, 18) est monté coulissant sur ladite tige principale (8, 16).

5. Instrument selon l'une quelconque des revendications précédentes, caractérisé en ce qu'un miroir (19) est monté coulissant sur ladite tige principale (16).

6. Instrument selon l'une quelconque des revendications 3 à 5, caractérisé en ce que ladite tige principale (8, 16) est graduée ce qui permet de repérer la distance entre les objets (10, 18) qui y sont montés coulissants et la monture (1).

7. Instrument selon l'une quelconque des revendications précédentes, caractérisé en ce que sur l'extrémité distale de la tige principale (8) est montée une tige secondaire (13) de préférence graduée, s'étendant dans une direction faisant un angle déterminé avec la direction de ladite tige principale.

8. Instrument selon la revendication 7, caractérisé en ce que ladite tige secondaire (13) est montée sur l'extrémité distale de la tige principale (8) de sorte à pouvoir effectuer une rotation d'angle déterminé, axée sur cette dernière.

9. Instrument selon la revendication 7, caractérisé en ce qu'un anneau gradué ou analogue (11) est fixé à l'extrémité proximale ou distale de ladite tige principale (8), pour permettre de mesurer l'angle que fait la direction de ladite tige secondaire (13) avec le plan vertical contenant ladite tige principale (8).

10. Instrument selon l'une quelconque des revendications 7 à 9, caractérisé en ce qu'un objet de repère (15), avantageusement lumineux, est monté à l'extrémité libre de ladite tige secondaire (13), un curseur (14) étant en outre monté coulissant sur celle-ci.

11. Instrument selon l'une quelconque des revendications 7 à 10, caractérisé en ce que ladite tige secondaire (22) s'étend d'un côté et d'autre d'un plan contenant la tige principale (21) et qui lui est perpendiculaire, l'une de ses extrémités (26) étant libre, tandis que l'autre (23) est reliée à l'extrémité distale (24) de la tige principale (21) par un bras (25).

12. Instrument selon la revendication 11, caractérisé en ce que ladite tige, secondaire (22) porte, à ses deux extrémités (23, 26), un objet de repère, un curseur (27) étant en outre monté coulissant sur celle-ci.

13. Instrument selon l'une quelconque des revendications précédentes, caractérisé en ce qu'outre ladite tige principale (8), d'autres tiges (29) partent en divergeant du pont central (2) de la monture, ces tiges accessoires (29) ayant une longueur telle qu'elles permettent de mettre en évidence la diplopie physiologique périphérique.

14. Instrument selon la revendication 13, caractérisé en ce que lesdites tiges accessoires (29) portent chacune au moins un repère (30), pour la représentation de la vision simultanée périphérique.

15. Instrument selon la revendication 13 ou 14, caractérisé en ce que lesdites tiges accessoires (29) sont orientables.

**Patentansprüche**

1. Gerät zum Prüfen, Messen und Behandeln von Anomalien des binokularen Sehens, mit einem Gestell (1), das herkömmlichen Brillengestellen entsprechen kann und eine mittlere Brücke (2) aufweist, die zum Aufsetzen auf der Nase der Person geeignet ist und zwei Halter (3) verbindet, die der Halterung optischer Gläser vor den Augen dienen, wobei an den Haltern (3), in an sich bekannter Weise, zwei Bügel (4) angelenkt sind, die, wenn sie aufgeklappt sind, den Kopf der Person einfassen und durch deren Ohren abgestützt werden können, dadurch gekennzeichnet, dass an der Vorderseite der erwähnten Brücke (2) eine erste Stange (8, 16) befestigt ist, die sich nach vorn erstreckt, d.h. in weitgehend entgegengesetzter Richtung zu der der erwähnten Bügel (4), wenn sie aufgeklappt sind, wobei die Stange eine Länge aufweist, die ausreicht, um ein physiologisches Doppeltsehen hervorzurufen, und dass die erwähnte erste Stange (8, 16) in der Weise an der erwähnten Brücke (2) des Gestells (1) drehbar gelagert ist, dass sie verschiedene gegenüber der Horizontalen geneigte Lagen wenigstens in einer vertikalen Sagittalebene der Person und eventuell in vertikalen Ebenen, die seitlich geneigt auf der einen oder anderen Seite dieser Sagittalebene verlaufen, einnehmen kann.

2. Gerät nach Anspruch 1, dadurch gekennzeichnet, dass das Gestell (1) ein Diopterlineal und/oder einen mit einer Gradeinteilung versehenen Kreis (11) oder Kreisbogen trägt, d.h. Vorrichtungen, die es gestatten, verschiedene Neigungswinkel der erwähnten ersten Stange (8, 16) in der erwähnten Sagittalebene oder des seitlichen Winkelabstandes der sie enthaltenden Vertikalebene relativ zu derselben Sagittalebene zu messen.

3. Gerät nach Anspruch 1 oder 2, dadurch gekennzeichnet, dass auf der erwähnten ersten Stange (8, 16) ein opakes Lineal (20), im wesentlichen nach unten gerichtet, verschiebbar gelagert ist.

4. Gerät nach einem der vorstehenden Ansprüche, dadurch gekennzeichnet, dass auf der erwähnten ersten Stange (8, 16) wenigstens ein Markierungsläufer (10, 18) verschiebbar gelagert ist.

5. Gerät nach einem der vorstehenden Ansprüche, dadurch gekennzeichnet, dass auf der erwähnten ersten Stange (16) ein Spiegel (19) verschiebbar gelagert ist.

6. Gerät nach einem der Ansprüche 3 bis 5, dadurch gekennzeichnet, dass die erwähnte erste Stange (8, 16) mit einer Skala versehen ist, so dass der Abstand der auf ihr verschiebbar gelagerten Gegenstände (10, 18) von dem Gestell (1) markiert werden kann.

7. Gerät nach einem der vorstehenden Ansprüche, dadurch gekennzeichnet, dass am distalen Ende der ersten Stange (8) eine zweite Stange (13) angebracht ist, die vorzugsweise mit einer Skala versehen ist und sich in einer Richtung erstreckt, die einen Winkel mit der Richtung der erwähnten ersten Stange einschliesst.

8. Gerät nach Anspruch 7, dadurch gekennzeichnet, dass die erwähnte zweite Stange (13) in der Weise an dem distalen Ende der ersten Stange (8) angebracht ist, dass sie um die Achse der letzteren eine Drehung um einen vorbestimmten Winkel ausführen kann.

9. Gerät nach Anspruch 7, dadurch gekennzeichnet, dass ein mit einer Gradeinteilung versehener Ring (11) o.dgl. am proximalen oder distalen Ende der erwähnten ersten Stange (8) befestigt ist, um den Winkel zwischen der Richtung der erwähnten zweiten Stange (13) und der Vertikalebene, in der die erwähnte erste Stange (8) liegt, messen zu können.

10. Gerät nach einem der Ansprüche 7 bis 9, dadurch gekennzeichnet, dass ein Markierungsobjekt (15), vorzugsweise ein beleuchtetes, am freien Ende der erwähnten zweiten Stange (13) angebracht und ein weiterer Läufer (14) auf der zweiten Stange verschiebbar gelagert ist.

11. Gerät nach einem der Ansprüche 7 bis 10, dadurch gekennzeichnet, dass die erwähnte zweite Stange (22) sich auf die eine und die andere Seite und senkrecht zu einer Ebene erstreckt, in der die erste Stange (21) liegt, wobei das eine ihrer Enden (26) frei, dagegen das andere (23) mit dem distalen Ende (24) der ersten Stange (21) durch einen Arm (25) verbunden ist.

12. Gerät nach Anspruch 11, dadurch gekennzeichnet, dass die erwähnte zweite Stange (22) an ihren beiden Enden (23, 26) ein Markierungsobjekt trägt und dass ferner ein Läufer (27) verschiebbar auf ihr gelagert ist.

13. Gerät nach einem der vorstehenden Ansprüche, dadurch gekennzeichnet, dass ausser der erwähnten Stange (8) weitere Stangen (29) divergierend von der mittleren Brücke (2) des Gestells abgehen und diese zusätzlichen Stangen (29) eine solche Länge aufweisen, dass sie es gestatten, das periphere physiologische Doppeltsehen erkennbar zu machen.

14. Gerät nach Anspruch 13, dadurch gekennzeichnet, dass die erwähnten zusätzlichen Stangen (29) jeweils wenigstens eine Markierung (30) zur Darstellung des peripheren gleichzeitigen Sehens tragen.

15. Gerät nach Anspruch 13 oder 14, dadurch gekennzeichnet, dass die erwähnten zusätzlichen Stangen (29) in ihrer Richtung einstellbar sind.

**Claims**

1. An instrument for the examination, measurement and treatment of binocular vision anomalies, of the type comprising a frame (1), which may be similar to that of conventional spectacles comprising a central bridge (2) adapted to bear on the subject's nose and connecting two optical glass supports (3) adapted to hold said optical glasses respectively in front of the eyes, two arms (4) being articulated, also in known manner, on said supports (3) and being adapted, when unfolded, to frame the subject's head, being supported by the subject's ears, characterised in that a main rod (8, 16) is fixed on the front surface of the bridge (2) and extends forwardly, i.e. in a general direction opposite to the direction of the arms (4) when the latter are in the unfolded position, said rod having a sufficient length to produce physiological diplopia, and in that said main rod (8, 16) is mounted rotatably on said bridge (2) of the frame (1) so as to be able to assume various inclined positions to the horizontal, at least in the subject's sagittal vertical plane and possibly in vertical planes inclined laterally on one side or the other of said sagittal plane.

2. An instrument according to claim 1, characterised in that the frame (1) bears an alidade and/or a graduated circle or arc of a circle (11), means adapted to allow measurement of the different angles of inclination of said main rod (8, 16) in said sagittal plane, or the angle of lateral spacing from the vertical plane containing it, with respect to said sagittal plane.

3. An instrument according to claim 1 or 2, characterised in that an opaque rule (20) extending downwardly in principle is mounted slidably on said main rod (8, 16).

4. An instrument according to any one of the preceding claims, characterised in that at least one reference cursor (10, 18) is mounted slidably on said main rod (8, 16).

5. An instrument according to any one of the preceding claims, characterised in that a mirror (19) is mounted slidably on said main rod (16).

6. An instrument according to any one of claims 3 to 5, characterised in that the said main rod (8, 16) is graduated, so that it is possible to log the distance between the objects (10, 18) mounted slidably thereon and the frame (1).

7. An instrument according to any one of the preceding claims, characterised in that a preferably graduated secondary rod (13) is mounted on the distal end of the main rod (8) and extends in a direction forming a given angle with the direction of said main rod.

8. An instrument according to claim 7, characterised in that the said secondary rod (13) is mounted on the distal end of the main rod (8) so as to allow a rotation through a given angle, said rotation being centred on the latter.

9. An instrument according to claim 7, characterised in that a graduated ring or the like (11) is fixed to the proximal or distal end of said main rod (8) to allow measurement of the angle between the direction of the said secondary rod (13) and the vertical plane containing said main rod (8).

10. An instrument according to any one of claims 7 to 9, characterised in that an advantageously luminous reference object (15) is mounted at the free end of said secondary rod (13), a cursor (14) also being mounted slidably thereon.

11. An instrument according to any one of claims 7 to 10, characterised in that the said secondary rod (22) extends on either side of a plane containing the main rod (21), and which is perpendicular thereto, one of its ends (26) being free while the other (23) is connected to the distal end (24) of the main rod (21) by an arm (25).

12. An instrument according to claim 11, characterised in that the two ends (23, 26) of said secondary rod (22) bear a reference object, a cursor (27) also being mounted slidably thereon.

13. An instrument according to any one of the preceding claims, characterised in that in addition to the said main rod (8) other rods (29) extend divergently from the central bridge (2) fo the frame, said accessory rods (29) having a length such as to show peripheral physiological diplopia.

14. An instrument according to claim 13, characterised in that the said accessory rods (29) each bear at least one reference (30) to represent the peripheral simultaneous vision.

15. An instrument according to claim 13 or 14, characterised in that the said accessory rods (29) are orientatable.

# Fig.1.

0 128 783

1/3

Fig.2.

Fig.3.

0 128 783

FIG.4 .

0 128 783

3/3